# EUROPEAN PATENT APPLICATION

(11) **EP 3 698 771 A1**
(43) Date of publication of application: **26.08.2020**
(21) Application number: 18868820.4
(22) Date of filing: 16.10.2018
(51) Int. Cl.: A61K 8/60, A61Q 5/00, A61Q 7/00, A61Q 19/00, A61Q 19/08

(54) **COSMETIC COMPOSITION COMPRISING NICOTINAMIDE MONONUCLEOTIDE**

(30) Priority: 16.10.2017 JP 2017199979
(71) Applicant: Tanaka, Megumi, Chigasaki-shi, Kanagawa 253-0054 (JP); Tanaka, Tsunemaru, Chigasaki-shi, Kanagawa 253-0054 (JP)
(72) Inventor: Tanaka, Megumi, Chigasaki-shi, Kanagawa 253-0054 (JP); Tanaka, Tsunemaru, Chigasaki-shi, Kanagawa 253-0054 (JP)
(74) Representative: IPAZ
(86) International application number: PCT/JP2018/038392
(87) International publication number: WO 2019/078177

(57) **Abstract**

PROBLEM: To provide a cosmetic composition that has a moisturizing effect that gives lasting moisture to a skin, an effect that prevents or improves aging symptoms, such as wrinkles and sags, and similar effect.

SOLUTION: Nicotinamide mononucleotide is contained in a cosmetic composition.

## Description

### TECHNICAL FIELD

The present invention relates to a cosmetic composition having a moisturizing effect that gives lasting moisture to a skin, an effect that prevents or improves aging symptoms, such as wrinkles and sags, and similar effect, specifically, relates to a cosmetic composition a containing nicotinamide mononucleotide.

### BACKGROUND ART

While skin moisture is kept by a water content inside a stratum corneum and a sebum barrier on a surface of skin, the skin loses the water content and is likely to be dried when the air is dry. In particular in the winter, since the coldness constricts blood circulation and reduces sebum on the surface of the skin, the water content of the skin is easily vaporized to make the skin further dry. An amount of production of moisturizing factor is gradually reduced with age, and therefore, aging also easily causes a dry skin. Therefore, recently, people have increasing awareness of skin care for preventing the dry skin and keeping the skin moist.

In fact, a recent survey that targeted women all around the country obtained a result of answers that the highest concern of women in skin care was "moisturizing," and the most important point or effect that they focus in selecting cosmetic products was a "moisturizing ability." This result is said to be a proof that many women are troubled by dry skins.

Under such circumstances, there are sold various cosmetic products added with a moisturizing function. Such cosmetic products are generally provided in a form of lotion, milky lotion, or cream. The lotion is used for supplementing water content and moisturizing factor in a skin. The milky lotion and the cream are usually used after the lotion to resupply the water content and the moisturizing factor in the skin, and play a role to protect a surface of the skin by forming a sebum barrier on the surface of the skin.

Conventionally, there are sold cosmetic products in which various kinds of moisturizing factors are contained in order to prevent a skin from getting dried and keep the skin moist, and as the moisturizing factors contained in general cosmetic products, for example, hyaluronic acid, collagen, elastin, ceramide, hydrogenated soybean lecithin, polyethylene glycol, a heparinoid, and a proteoglycan are well known.

Cosmetic compositions containing new moisturizing factors other than ones described above are actively developed. For example, there has been reported a cosmetic composition that contains galactomannan, as a plant polysaccharide having a high moisturizing effect, and being low in price and high in safety, or a cross-linked product formed of its derivative as a moisturizing factor (Patent Document 1).

Other than this, there has been reported a cosmetic composition that contains a skin lightening agent and a plant-derived polyamine-containing extract derived from at least one kind of ingredients selected from a group consisting of a soybean seed, a soybean germ, a soybean embryo, a soybean bud, a wheat seed, a wheat germ, a wheat embryo, a wheat bud, a soy milk, and a soy pulp, which have, for example, a moisturizing effect, a collagen generation promoting effect, a hyaluronic acid generation promoting effect, a cell activating effect, an antioxidant effect, an anti-aging effect, a hair restoration effect, and a skin-roughness improving effect (Patent Document 2).

As another example, there has been reported a cosmetic composition that contains a combination of at least two osmolytes selected from a group consisting of taurine or its derivative, inositol, betaine, and trehalose as a composition that recovers, maintain, or increases moisture in skin, and/or protects the skin from various types of stresses, and/or prevents or delays appearance of sign of skin aging, or weakens the effect, or promotes long life of cells or tissues (Patent Document 3).

As described above, having a moisturizing function is an important factor for a cosmetic product to meet the needs of consumers. However, when a high concentration of humectant is contained in order to improve the function, the moisturizing factor used in conventional cosmetic products has increased stickiness, or the moisturizing factor itself keeps water content and gives moisture, and therefore, the moisturizing effect does not last. This requires a frequent application to the skin, thereby causing a problem that moisturizing properties are not inherently improved.

Recently, in addition to the moisturizing function, other functions are also attracting consumers' interests. For example, regarding functional cosmetic products, which are cosmetic products including an aspect of therapy in ordinary skin care by containing ingredients used in medicinal products in addition to ingredients as basic skin care products, an effect that prevents or improves aging symptoms (an anti-aging effect), such as wrinkles and sags, is highly attracting interests as a result of reflecting the aging society, besides a skin lightening effect, a sunscreen effect, and the like. Therefore, the development of the cosmetic product that have a combination of these effects is anticipated.

Patent Document 1: JP-A-2004-217590
Patent Document 2: JP-A-2009-234939
Patent Document 3: JP-A-2010-150258

### DISCLOSURE OF THE INVENTION

### PROBLEMS TO BE SOLVED BY THE INVENTION

It is an object of the present invention to provide a cosmetic composition that has a moisturizing effect that gives lasting moisture to a skin, an effect that prevents or improves aging symptoms, such as wrinkles and sags, and similar effect.

### SOLUTIONS TO THE PROBLEMS

The inventor seriously studied in order to solve the above-described problems, and found that containing a nicotinamide mononucleotide as an intermediate metabolite involved in biosynthesis of a coenzyme nicotinamide adenine dinucleotide (NAD) obtains a lasting moisturizing effect and the like. Thus, the present invention was completed.

The present invention is as follows.
[1] A cosmetic composition that contains a nicotinamide mononucleotide.
[2] The cosmetic composition according to [1] where the nicotinamide mononucleotide has an amount of 0.0001 to 40 mass% of a total composition.
[3] The cosmetic composition according to [1] or [2] where the nicotinamide mononucleotide has a purity of 90% or more.
[4] The cosmetic composition according to any one of [1] to [3] where the cosmetic composition is used for improving a moisturizing property of skin.
[5] The cosmetic composition according to any one of [1] to [3] where the cosmetic composition is used for preventing or improving an aging symptom of skin.
[6] The cosmetic composition according to any one of [1] to [3] where the cosmetic composition is used for promoting a new hair growth or a hair restoration.
[7] The cosmetic composition according to any one of [1] to [3] where the cosmetic composition is used for preventing or improving a macule and a freckle of skin.
[8] The cosmetic composition according to any one of [1] to [3] where the cosmetic composition is used for preventing or improving a rough skin.
[9] The cosmetic composition according to any one of [1] to [3] where the cosmetic composition is used for reducing dandruff and an itch of scalp.
[10] The cosmetic composition according to any one of [1] to [3] where the cosmetic composition is used for preventing trichorrhexis, a hair breakage, and split ends.
[11] The cosmetic composition according to any one of [1] to [10] where the cosmetic composition is used as a cosmetic product or a quasi-drug.

### EFFECTS OF THE INVENTION

The cosmetic composition according to the present invention improves moisturizing properties by giving lasting moisture to a skin, and further, provides an effect, such as preventing or improving aging symptoms, such as wrinkles and sags.

### BRIEF DESCRIPTION OF THE DRAWINGS

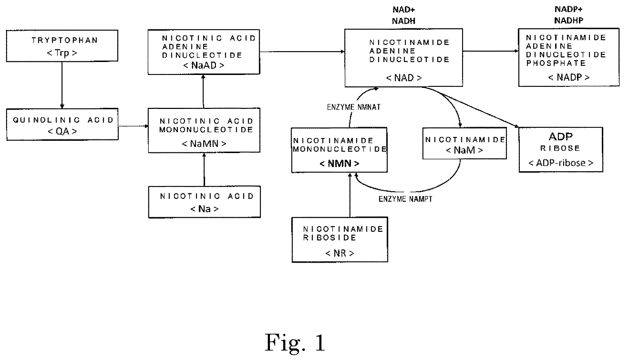

Fig. 1 is an explanatory drawing illustrating a metabolic pathway involved in niacin (generic term of nicotinamide and nicotinic acid).

### DESCRIPTION OF PREFERRED EMBODIMENTS

A cosmetic composition according to the present invention contains a nicotinamide mononucleotide, and containing this ensures providing a lasting moisturizing effect and the like. The detailed reason why containing the nicotinamide mononucleotide provides such an effect is currently examined. However, it is considered to be one reason that the nicotinamide mononucleotide promotes a "sirtuin" typified by NAD⁺ dependent deacetylases Sirt1 and Sirt3, and consequently, metabolism in vivo is activated. This is based on a new mechanism of action totally different from that of the conventional moisturizing factor in which the moisturizing factor itself keeps water content. Specifically, the nicotinamide mononucleotide is absorbed as soon as being applied to the outer layer of skin. This increases an amount of the nicotinamide mononucleotide in cells of the skin and it is metabolized to increase an amount of the NAD, thereby activating the sirtuin. The activation of the sirtuin of the skin promotes generating a natural moisturizing factor, and as a result, a skin moisturizing effect and an aging prevention and improvement effect can be obtained. The following describes the present invention in detail.

The nicotinamide mononucleotide (chemical formula: C₁₁H₁₅N₂O₈P) contained in the cosmetic composition according to the present invention is a compound expressed with a structural formula [Chem. 1] below. The nicotinamide mononucleotide is generally referred to as nicotinamide mononucleotide (NMN), and known as an intermediate metabolite involved in a biosynthesis of coenzyme NAD⁺. The nicotinamide mononucleotide is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to Fig. 1. Fig. 1 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

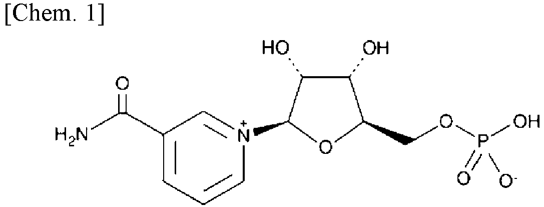

As illustrated in Fig. 1, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN) (de novo pathway). Meanwhile, when the nicotinic acid (Na) is the starting material, the nicotinic acid is directly converted into the NaMN Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD (Salvage pathway). Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730). Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available and those commercial products can be purchased for use.

In the cosmetic composition according to the present invention, a content of the nicotinamide mononucleotide may be appropriately determined depending on a purpose of use, a frequency of use, an age of a target user, a condition, a weight, an age, and the like. However, the content is preferably 0.0001 to 40 mass% of the total composition, more preferably 0.0001 to 20 mass%, and further preferably 0.001 to 10 mass%. When the content is less than 0.0001 mass%, it possibly fails to provide the effect of the present invention, and when the content is more than 40 mass%, it is disadvantageous in the aspect of cost.

The nicotinamide mononucleotide contained in the cosmetic composition according to the present invention is a purified product that contains a few impurities, especially, preferably its purity is 90% or more, and further preferably its purity is 95% or more. When the purity is less than 90%, a bad smell possibly occurs, or the effect of the nicotinamide mononucleotide is possibly reduced to fail to sufficiently provide the effect of the present invention.

While the purity of the nicotinamide mononucleotide contained in the cosmetic composition according to the present invention is preferably 90% or more as described above, the purity (mass ratio) is defined as a value obtained by subtracting the impurities other than the nicotinamide mononucleotide from 100% in anhydrous terms. Accordingly, the purity of the nicotinamide mononucleotide can be obtained with a formula: nicotinamide mononucleotide purity (%) = 100 - impurities other than nicotinamide mononucleotide (%). Here, these impurities include, as illustrated in Fig. 1, metabolites excluding the nicotinamide mononucleotide involved in the NAD metabolic pathway, especially, the nicotinamide and the nicotinamide adenine dinucleotide. When the nicotinamide mononucleotide used in the present invention contains a foreign element such as the above-described metabolite involved in the NAD metabolic pathway, for example, the absorption of the nicotinamide mononucleotide into living cells possibly reduces, resulting in the reduction of the effect of the present invention. A quantitative determination of the above-described impurities involved in the NAD metabolic pathway is performed with an absolute calibration curve method using a standard sample where a test solution of dried nicotinamide mononucleotide powder is poured into an HPLC apparatus, and a peak area of an obtained chromatograph is obtained (vertical axis: peak area, horizontal axis: concentration). Since the use of the peak height ensures the quantitative determination with high accuracy in a case of a trace substance, the apparatus to be used is appropriately chosen according to the characteristics of the apparatus. Separated substances are identified based on retention times.

The cosmetic composition according to the present invention can be applied on a face, hand, and the like to be used to give moisture to the skin and improve moisturizing properties of the skin to last. One of the features of the present invention is that the moisturizing effect lasts. The application of the present invention increases a moisture amount in the skin to improve the moisturizing properties of the skin, thereby improving dryness, tension, elasticity, softness, and the like of the skin. The reason why the improvement in the moisturizing properties of the skin is provided by the present invention is considered that the nicotinamide mononucleotide affects to enhance the production of natural moisturizing factors and keratin intercellular lipids, and promotes the generation of the hyaluronic acid. Thus, the nicotinamide mononucleotide can be used as a humectant as it is, and can be used for manufacturing a humectant.

The cosmetic composition according to the present invention can be applied on a face, hand, and the like to be used to prevent and improve aging symptoms of the skins. Here, the "aging symptoms of skin" mainly means an occurrence of wrinkles and sags, a loss of skin tension, and the like, caused by reduced elasticity of the skin in association with aging. The reason why the aging symptoms of the skin are prevented and improved by the present invention is considered that, for example, the nicotinamide mononucleotide activates a weakened skin metabolic function and promotes a collagen formation. Thus, the nicotinamide mononucleotide can be used as an agent for preventing or improving skin aging as it is, and can be used for manufacturing an agent for preventing or improving skin aging.

The cosmetic composition according to the present invention can be applied on a scalp and hair to be used to provide a new hair growth and hair restoration effect. The reason why the new hair growth and hair restoration effect is provided by the present invention is considered that the nicotinamide mononucleotide activates hair matrix cellssplit. Thus, the nicotinamide mononucleotide can be used as a new hair growth agent and a hair restoring agent as it is, and can be used for manufacturing a new hair growth agent and a hair restoring agent.

The cosmetic composition according to the present invention can be used for obtaining a skin lightening effect that prevents a generation and hyperpigmentation of melanin caused by sunburn and the like, and prevents or improves macules and freckles of the skin. The reason why the skin lightening effect is provided by the present invention is considered that the nicotinamide mononucleotide promotes discharging the melanin and the like. Thus, the nicotinamide mononucleotide can be used as a skin lightening agent as it is, and can be used for manufacturing a skin lightening agent.

The cosmetic composition according to the present invention can be used for preventing and improving a rough skin caused by an effect from outside and an effect of internal environment. It is further effective to appropriately contain an anti-inflammatory agent, a vitamin, a hormone, a plant extract, a fungicide, an oily component, and the like depending on a cause of the rough skin. The reason why the preventive and improving effect is provided by the present invention is considered that the nicotinamide mononucleotide improves a function of a sirtuin in skin tissues and the like. Thus, the nicotinamide mononucleotide can be used as a preventive or improving agent for rough skin as it is, and can be used for manufacturing a preventive or improving agent for rough skin.

The cosmetic composition according to the present invention can be used for reducing dandruff and an itch of scalp. The reason why the reducing effect of the dandruff and itch of the scalp is provided by the present invention is considered that the nicotinamide mononucleotide affects the scalp to reduce an inflammation of the scalp.

The cosmetic composition according to the present invention can be used for preventing trichorrhexis, a hair breakage, and split ends. The reason why the preventive effect of the trichorrhexis, hair breakage, and split ends is provided by the present invention is considered that the nicotinamide mononucleotide affects the hair to reinforce cuticles, and promotes its production.

The cosmetic composition according to the present invention can be used as a cosmetic product or a quasi-drug. Here, the "cosmetic product" is defined as a product that aims to be used in a way of inunction, spraying, or another way similar to those with respect to the body in order to clean a human body, beautify, make more attractive, change physical appearance, or maintain the skin or hair healthy, and a product having a mild effect on a human body. The "quasi-drug" is a product that is used to aim for preventing any discomfort including nausea, or bad breath or body odor, preventing heat rash, erosion, and the like, or preventing hair loss, restoring hair or removing hair, and is a product having a mild effect on a human body.

The cosmetic product or the quasi-drug according to the present invention is applied on a skin, preferably, approximately one to five times a day and used for approximately a few days to a few months until a desired effect is obtained. Dosage of cosmetic product or quasi-drug according to the present invention may be appropriately determined corresponding to a content of the nicotinamide mononucleotide, desired effect, form of cosmetic product or quasi-drug, condition of target user, weight, age, and the like.

The cosmetic composition according to the present invention can be provided in various kinds of forms. Specifically, the forms can be: skin care cosmetics, such as a facial cleanser (e.g. a facial soap, a toilet soap, a face wash, and a face wash cream), a make-up remover (e.g. a cleansing cream), a cleansing cosmetic, a lotion, a serum, a facial mask, a skin conditioning cosmetic, a protection milky lotion (a moisture lotion), a protection cream (a moisture cream), a protection cosmetic, a skin lightening cosmetic, and a ultraviolet-ray protecting cosmetic; makeup cosmetics, such as a foundation, a powder (a face powder), a makeup base, a base makeup cosmetic, a lipstick, an eye makeup (e.g. an eye shadow and an eye liner), a blush (a cheek color), a nail enamel (e.g. a nail enamel remover), and a point makeup cosmetic; hair care cosmetics, such as a shampoo, a hair rinse, a hair treatment, a hair conditioner, a hair wash cosmetic, a hairdressing cosmetic, a hair foam, a hair mousse, a hair spray, a hair styling gel, a hair wax, a hair oil, a pomade, a tique, a hair liquid, a hair styling liquid, a permanent wave agent, a hair dye, a decolorant, a hair dye remover, and a hair restoring agent; body care cosmetics, such as a bath soap, a body shampoo, a hand soap, a body cleansing cosmetic, a deodorant cosmetic, and a bath additive; toothpastes; and fragrance cosmetics, such as a perfume, an eau de cologne, and an eau de toilette.

The cosmetic composition according to the present invention appropriately contains, for example, a base material that constitutes a base of the cosmetic composition, a quality maintaining material necessary for maintaining the quality, and a sensuous feature adding material for adding a color and a fragrance, depending on a purpose of use and the like. Specifically, the examples include: oily components, such as hydrocarbons, a vegetable oil, an animal oil, a higher alcohol, a fatty acid, a fatty acid ester, a silicone oil, polyhydric alcohols, a polyhydric alcohol ether, an alkyl glyceryl ether, and a fluorine oil; anionic, cationic, zwitterionic, and nonionic surfactants; high polymeric substances, such as polyhydric alcohols, saccharides, a water-soluble biopolymer (e.g. hyaluronic acid, collagen, elastin, albumin, casein sodium, and pyrrolidone carboxylic acid), a natural moisturizing factor (e.g. PCA-Na), amino acids, a moisturizing factor such as urea, a water-soluble polymer (e.g. carboxymethyl cellulose, hydroxyethyl cellulose, hydroxypropyl cellulose, carrageenan, guar gum, xanthan gum, cellulose gum, poval, PVP, and cationized cellulose), a biopolymer (e.g. sodium hyaluronate, collagen, and albumin), a film-forming polymer (e.g. poval, PVP, nitrocellulose, alkyd resin, shellac, chitosan, xanthan gum, dimethicone, and diphenyl dimethicone), a polymer powder (powder polyethylene, polyamide, nylon powder, polyethylene terephthalate/polymethyl methacrylate laminated powder), an inorganic high polymer (e.g. silica, zinc white, zinc oxide, mica, hydrated aluminum potassium silicate, isinglass, sericite, hydrated aluminum silicate, kaolin, china clay, talc, bentonite, titanium oxide, and calcium carbonate); solvents, such as a viscosity improver, an organic solvent (e.g. ethyl acetate, ethanol, propanol, ethylene glycol, and glycerin); color materials, such as an organic synthetic dye, a natural dye, an inorganic pigment (e.g. silica, zinc oxide, bronze mica, kaolin, talc, bentonite, iron oxide, titanium oxide, chromium oxide, and calcium oxide), a pearl pigment (an aluminum powder, titanium oxide, iron oxide, mica, and argentine), a polymer powder, a glitter pigment, an extender pigment (e.g. bronze mica, silica, talc, and calcium carbonate), a white pigment, and a coloring pigment; natural fragrances, such as jasmone, citral, borneol, d-limonene, eugenol, a natural oil, and a natural extract; synthetic fragrances, such as benzyl benzoate, methyl benzoate, vanillin, ethylvanillin, undecalactone, coumarin, cinnamaldehyde, cinnamyl alcohol, ethyl cinnamate, geraniol, citronellal, salicylate ester, benzyl alcohol, methylacetophenone, and phenethyl alcohol; ultraviolet inhibitors, such as ethyl PABA, oxybenzone-1, oxybenzone-6, homosalate, t-butyl methoxydibenzoylmethane, and oryzanol; UV scattering agents, such as titanium oxide and zinc white; preservatives, such as benzoic acid, salicylic acid, ethylparaben, butylparaben, sorbic acid, phenoxyethanol, sodium dehydroacetate, and ester parahydroxybenzoate; fungicides, such as benzalkonium chloride, benzethonium chloride, quaternary ammonium salt, chlorohexidine, zinc pyrithione, propylene glycol, and hinokitiol; antioxidizing agents, such as dibutylhydroxytoluene, butylhydroxyanisole, and tocopherol; chelating agents, such as ethylenediamine tetraacetate, citric acid, and phytic acid; pH adjusters, such as ethanolamine, DEA, TEA, sodium hydroxide, citric acid, tartaric acid, sodium acetate, glycolic acid, and lactic acid; oxidants, such as hydrogen peroxide and sodium bromate; reductants, such as mercaptoacetic acid, DL-cysteine, and sodium sulfite; penetrants, such as benzyl alcohol; and solvents, such as ethyl acetate, butyl acetate, ethanol, and water.

Furthermore, the cosmetic composition according to the present invention can contain components having a mild pharmacological effect contained in ordinary cosmetic products, cosmeceuticals, quasi-drugs, and the like in a range within which the effect of the present invention is not inhibited. Examples of such a component specifically includes: skin lightening agents, such as vitamin C and its derivative; blood circulation promoters, such as tocopherol, a chili pepper extract, a swertia japonica extract, a garlic extract, and a panax ginseng root extract; hair restoring agents, such as a hair matrix cell activator agent, an antiandrogen agent, an anti-inflammatory agent, an anti-dandruff agent, and an antipruritic; rough skin prevention agents, such as an anti-inflammatory agent, a vitamin, hormones, and plant extracts; wrinkle prevention agents, such as an antioxidant agent (e.g. vitamin C, tocopherol, carotene, epicatechin, and glycyrrhetinic acid), a cell activator agent, a collagenase inhibitor, and an elastase inhibitor; acne agents, such as a sebum secretion inhibitor, a stratum corneum delamination/dissolution agent, a fungicide, and an anti-inflammatory agent (e.g. allantoin, aminocaproic acid, calcium pantothenate, provitamin B₅, glycyrrhetinic acid, and lysozyme chloride); dandruff/itch agents; underarm odor preventing agents, such as a antiperspirant substance (aluminum chlorohydrate, aluminum chlorohydroxy allantoinate, aluminum potassium sulfate, zinc sulfate, and zinc para-phenolsulfonate), a moisture absorber (e.g. talc, zinc oxide, a fine silica powder, and porous polymer); astringents, such as an organic acid (e.g. citric acid and succinic acid), and a metal salt (e.g. zinc oxide, zinc sulfate, and allantoin dihydroxyaluminum); amino acids; and vitamins.

The cosmetic composition according to the present invention may be manufactured in a known method generally used in the cosmetics and pharmaceutical field by appropriately selecting the nicotinamide mononucleotide and other ingredients described above and using the appropriate amount of them depending on a purpose of use and a configuration.

### [Working Example]

The following describes the present invention in detail based on the working examples, while the present invention is not limited by these working examples.

### Production Example 1

With a usual method, nicotinamide mononucleotide, sodium hyaluronate, a PEG-20 sorbitan cocoate, ethylhexylglycerin, potassium hydroxide, water, butylene glycol, glycerin, and pentylene glycol were uniformly mixed to manufacture a lotion (a present invention product 1, "FACE RICH," product name, manufactured by SHINKOWA PHARMACEUTICAL Co., Ltd.) that contained 0.1 mass% of the nicotinamide mononucleotide.

### Production Example 2

With a usual method, nicotinamide mononucleotide, sodium hyaluronate, xanthan gum, ethylhexylglycerin, potassium hydroxide, water, butylene glycol, glycerin, and pentylene glycol were uniformly mixed to manufacture a serum (a present invention product 2, "FACE RICH SKIN ESSENCE," product name, manufactured by SHINKOWA PHARMACEUTICAL Co., Ltd.) containing 1.0% of the nicotinamide mononucleotide.

### Production Example 3

With a usual method, nicotinamide mononucleotide, a glyceryl stearate, stearic acid PEG-10, sorbitan stearate, a horse oil, dimethicone, behenyl alcohol, cetearyl glucoside, a jojoba seed oil, an olive fruit oil, EDTA-2Na, tocopherol, polyglyceryl-10 pentastearate, ethylhexylglycerin, sodium stearoyl lactate, potassium hydroxide, water, a rice bran oil, caprylic/capric triglyceride, glyceryl, glycerin, pentylene glycol, dipropylene glycol, cetearyl alcohol, and glyceryl stearate were uniformly mixed to manufacture a cream (a present invention product 3, "FACE RICH SKIN CREAM," product name, manufactured by SHINKOWA PHARMACEUTICAL Co., Ltd.) containing 0.1 mass% of the nicotinamide mononucleotide.

### Working Example 1

27 subjects (8 males, 19 females, age 28 to 70) used the lotion (the present invention product 1) manufactured in Production Example 1 continuously for two months. The subjects performed self-evaluations on skin moisturizing properties (dryness, tension, elasticity, and softness of skin) with criteria indicated in Table 1 at each of two weeks, one month, and two months after the start of using. Table 1 indicates the result of the evaluated number of people for each criterion.

### Working Example 2

27 subjects (8 males, 19 females, age 28 to 70) used the lotion (the present invention product 1) manufactured in Production Example 1 continuously for two months. The subjects performed self-evaluations on skin aging symptoms (an occurrence of wrinkles and sags, and a loss of skin tension) with criteria indicated in Table 1 at each of two weeks, one month, and two months after the start of using. Table 1 indicates the result of the evaluated number of people for each criterion.

### Working Example 3

27 subjects (8 males, 19 females, age 28 to 70) used the lotion (the present invention product 1) manufactured in Production Example 1 continuously for two months. The subjects performed self-evaluations on macules and freckles of skin with criteria indicated in Table 1 at each of two weeks, one month, and two months after the start of using. Table 1 indicates the result of the evaluated number of people for each criterion.

### Working Example 4

27 subjects (8 males, 19 females, age 28 to 70) used the lotion (the present invention product 1) manufactured in Production Example 1 continuously for two months. The subjects performed self-evaluations on rough skin with criteria indicated in Table 1 at each of two weeks, one month, and two months after the start of using. Table 1 indicates the result of the evaluated number of people for each criterion.

**[Table 1]**

| | | Symptom got worse | Effect was not especially felt | Slightly improved | Improved | Considerably improved |
|---|---|---|---|---|---|---|
| Working Example 1 | Two weeks later | - | 6 | 13 | 7 | 1 |
| | One month later | - | 3 | 15 | 8 | 1 |
| | Two months later | - | - | 11 | 14 | 2 |
| Working Example 2 | Two weeks later | - | 5 | 17 | 5 | - |
| | One month later | - | 1 | 19 | 6 | 1 |
| | Two months later | - | - | 15 | 11 | 1 |
| Working Example 3 | Two weeks later | - | 4 | 14 | 8 | 1 |
| | One month later | - | 1 | 14 | 11 | 1 |
| | Two months later | - | - | 11 | 14 | 2 |
| Working Example 4 | Two weeks later | - | 4 | 20 | 3 | - |
| | One month later | - | 4 | 16 | 7 | - |
| | Two months later | - | 2 | 15 | 9 | 1 |

### [Evaluation Result]

As seen from the results indicated in Table 1, the present invention product 1 was confirmed to be effective in improving the skin moisturizing properties, the skin aging symptoms, the skin macules and freckles, and the rough skin.

### Working Example 5

30 subjects (11 males, 19 females, age 33 to 79) used the serum (the present invention product 2) manufactured in Production Example 2 continuously for two months. The subjects performed self-evaluations on skin moisturizing properties (dryness, tension, elasticity, and softness of skin) with criteria indicated in Table 2 at each of two weeks, one month, and two months after the start of using. Table 2 indicates the result of the evaluated number of people for each criterion.

### Working Example 6

30 subjects (11 males, 19 females, age 33 to 79) used the serum (the present invention product 2) manufactured in Production Example 2 continuously for two months. The subjects performed self-evaluations on skin aging symptoms (an occurrence of wrinkles and sags, and a loss of skin tension) with criteria indicated in Table 2 at each of two weeks, one month, and two months after the start of using. Table 2 indicates the result of the evaluated number of people for each criterion.

### Working Example 7

30 subjects (11 males, 19 females, age 33 to 79) used the serum (the present invention product 2) manufactured in Production Example 2 continuously for two months. The subjects performed self-evaluations on macules and freckles of skin with criteria indicated in Table 2 at each of two weeks, one month, and two months after the start of using. Table 2 indicates the result of the evaluated number of people for each criterion.

### Working Example 8

30 subjects (11 males, 19 females, age 33 to 79) used the serum (the present invention product 2) manufactured in Production Example 2 continuously for two months. The subjects performed self-evaluations on rough skin with criteria indicated in Table 2 at each of two weeks, one month, and two months after the start of using. Table 2 indicates the result of the evaluated number of people for each criterion.

**[Table 2]**

| | | Symptom got worse | Effect was not especially felt | Slightly improved | Improved | Considerably improved |
|---|---|---|---|---|---|---|
| Working Example 5 | Two weeks later | - | 9 | 18 | 3 | - |
| | One month later | - | 2 | 17 | 10 | 1 |
| | Two months later | - | - | 10 | 17 | 3 |
| Working Example 6 | Two weeks later | - | 11 | 17 | 2 | - |
| | One month later | - | 6 | 18 | 6 | - |
| | Two months later | - | 1 | 17 | 12 | - |
| Working Example 7 | Two weeks later | - | 12 | 13 | 3 | 2 |
| | One month later | - | 2 | 21 | 5 | 2 |
| | Two months later | - | 1 | 15 | 9 | 5 |
| Working Example 8 | Two weeks later | - | 7 | 18 | 4 | - |
| | One month later | - | 3 | 14 | 12 | - |
| | Two months later | - | 1 | 11 | 13 | 4 |

### [Evaluation Result]

As seen from the results indicated in Table 2, the present invention product 2 was confirmed to be effective in improving the skin moisturizing properties, the skin aging symptoms, the skin macules and freckles, and the rough skin.

### Working Example 9

30 subjects (11 males, 19 females, age 33 to 79) used the cream (the present invention product 3) manufactured in Production Example 3 continuously for two months. The subjects performed self-evaluations on skin moisturizing properties (dryness, tension, elasticity, and softness of skin) with criteria indicated in Table 3 at each of two weeks, one month, and two months after the start of using. Table 3 indicates the result of the evaluated number of people for each criterion.

### Working Example 10

30 subjects (11 males, 19 females, age 33 to 79) used the cream (the present invention product 3) manufactured in Production Example 3 continuously for two months. The subjects performed self-evaluations on skin aging symptoms (an occurrence of wrinkles and sags, and a loss of skin tension) with criteria indicated in Table 3 at each of two weeks, one month, and two months after the start of using. Table 3 indicates the result of the evaluated number of people for each criterion.

### Working Example 11

30 subjects (11 males, 19 females, age 33 to 79) used the cream (the present invention product 3) manufactured in Production Example 3 continuously for two months. The subjects performed self-evaluations on macules and freckles of skin with criteria indicated in Table 3 at each of two weeks, one month, and two months after the start of using. Table 3 indicates the result of the evaluated number of people for each criterion.

### Working Example 12

30 subjects (11 males, 19 females, age 33 to 79) used the cream (the present invention product 3) manufactured in Production Example 3 continuously for two months. The subjects performed self-evaluations on rough skin with criteria indicated in Table 3 at each of two weeks, one month, and two months after the start of using. Table 3 indicates the result of the evaluated number of people for each criterion.

**[Table 3]**

| | | Symptom got worse | Effect was not especially felt | Slightly improved | Improved | Considerably improved |
|---|---|---|---|---|---|---|
| Working Example 9 | Two weeks later | - | 14 | 20 | 11 | 1 |
| | One month later | - | 8 | 19 | 14 | 5 |
| | Two months later | - | 3 | 21 | 15 | 7 |
| Working Example 10 | Two weeks later | - | 17 | 18 | 11 | - |
| | One month later | - | 6 | 22 | 15 | 3 |
| | Two months later | - | 2 | 24 | 14 | 6 |
| Working Example 11 | Two weeks later | - | 12 | 24 | 8 | 2 |
| | One month later | - | 6 | 23 | 11 | 6 |
| | Two months later | - | 3 | 23 | 12 | 8 |
| Working Example 12 | Two weeks later | - | 15 | 19 | 10 | 2 |
| | One month later | - | 3 | 26 | 12 | 5 |
| | Two months later | - | 1 | 25 | 13 | 7 |

### [Evaluation Result]

As seen from the results indicated in Table 3, the present invention product 3 was confirmed to be effective in improving the skin moisturizing properties, the skin aging symptoms, the skin macules and freckles, and the rough skin.

## Claims

1. A cosmetic composition that contains a nicotinamide mononucleotide.

2. The cosmetic composition according to claim 1, wherein
the nicotinamide mononucleotide has an amount of 0.0001 to 40 mass% of a total composition.

3. The cosmetic composition according to claim 1 or 2, wherein
the nicotinamide mononucleotide has a purity of 90% or more.

4. The cosmetic composition according to any one of claims 1 to 3, wherein
the cosmetic composition is used for improving a moisturizing property of skin.

5. The cosmetic composition according to any one of claims 1 to 3, wherein
the cosmetic composition is used for preventing or improving an aging symptom of skin.

6. The cosmetic composition according to any one of claims 1 to 3, wherein
the cosmetic composition is used for promoting a new hair growth or a hair restoration.

7. The cosmetic composition according to any one of claims 1 to 3, wherein
the cosmetic composition is used for preventing or improving a macule and a freckle of skin.

8. The cosmetic composition according to any one of claims 1 to 3, wherein
the cosmetic composition is used for preventing or improving a rough skin.

9. The cosmetic composition according to any one of claims 1 to 3, wherein
the cosmetic composition is used for reducing dandruff and an itch of scalp.

10. The cosmetic composition according to any one of claims 1 to 3, wherein
the cosmetic composition is used for preventing trichorrhexis, a hair breakage, and split ends.

11. The cosmetic composition according to any one of claims 1 to 10, wherein
the cosmetic composition is used as a cosmetic product or a quasi-drug.
